# EUROPEAN PATENT APPLICATION

(11) **EP 2 014 295 A2**
(43) Date of publication of application: **14.01.2009**
(21) Application number: 08009684.5
(22) Date of filing: 28.05.2008
(51) Int. Cl.: A61K 36/758, A61K 36/9066, A61K 36/28, A61K 36/185, A61P 15/02

(54) **Topical compositions for the prevention and treatment of inflammatory and/or infective conditions of the genital area**

(30) Priority: 04.06.2007 IT MI20071136
(71) Applicant: Velleja Research SRL, 29010 Pontenure (PC) (IT)
(72) Inventor: Callegari, Alberto, 29010 Pontenure (PC) (IT); Speroni, Maurizio, 29010 Pontenure (PC) (IT)
(74) Representative: Minoja, Fabrizio

(57) **Abstract**

The present invention relates to topical compositions containing *Zanthoxylum bungeanum* extract, 18-beta glycyrrhetic acid, *Matricaria chamomilla* essential oil, *Melaleuca alternifolia* essential oil, *Curcuma longa* or curcumin extract and lactic or propionic acid, for the prevention and treatment of inflammatory and/or infective conditions affecting mainly the genital area, in particular vaginosis, vaginitis and vulvo-vaginitis, also those which are recurring.

## Description

The present invention relates to topical compositions for the prevention and treatment of inflammatory and/or infective conditions mainly localised in the genital area, in particular vaginosis, vaginitis and vulvo-vaginitis, even those that are recurring.

### Background of the invention

Infections of the female genital apparatus, vaginosis, vaginitis and vulvo-vaginitis, are one of the most widespread gynecological problems, whose incidence seems to be increasing.

Even if the causes are different and numerous, and not always of infectious origin, the majority of diagnoses identifies:
1) bacterial vaginosis, i.e. polymicrobial syndromes characterized by a radical modification of the vaginal ecosystem consisting, in particular, in the replacement of the normal lactobacillar flora by pathogens (mainly anaerobes), very often caused or complicated by *Gardnerella* infections;
*2) Candida* vaginitis and vulvo-vaginitis;
*3) Trichomonas vaginalis* vaginitis.

Bacterial vaginosis is the most common vaginal and/or vulvo-vaginal infection among women in the fertile age. There are many predisposing factors for these alterations of the bacterial vaginal flora, for example: incorrect hygiene, intrauterine diaphragms, immune deficiencies, diabetes, sexual activity with more partners. The most typical symptom of bacterial vaginosis is abundant, smelly and greyish vaginal loss; itching, vaginal burning and pain during sexual intercourse can rarely be experienced. Pathogenic bacteria, generally *Gardnerella vaginalis,* which almost completely replace the normal lactobacillar flora, are one of the most common causes of vaginosis.

Even if vaginal secretion is increased in vaginosis, it is not possible to demonstrate the presence of an actual inflammatory state; instead, vaginitis appears with symptoms like burning, leucorrhoea, itching and inflammation of the mucosa, which are very tiresome, dramatically affect the patients' life quality and are often recurring.

Vaginitis is an inflammation of the vagina which can affect women of any age; it is called vulvo-vaginitis when it affects also the vulva, i.e. external genitals. In vulvo-vaginitis, fungi of the *Candida* genus often play a key role as infective agents.

These inflammations can be episodic, recurring (i.e. they appear again after treatment) and in some cases even chronic.

As already mentioned, a fungus, *Candida albicans,* which is usually present in the body, but which can become pathogenic under certain circumstances, is one of the main causes of the disorder. Candida vaginitis and vulvo-vaginitis involve: leukorrhoea, i.e. whitish and lumpy vaginal secretion with a characteristic consistency; itching, burning and, sometimes, pain at urinating; sexual transmission is possible.

Another pathogenic agent very often involved in vaginitis and/or vulvo-vaginitis, either episodic or recurring, is a protozoan, known as *Trichomonas vaginalis,* which is transmitted through sexual intercourse and which causes abundant, smelly and yellow-greenish secretion frequently associated to intense itching, pain during sexual intercourse and trouble at urinating.

Reference and consolidated therapies (either local or systemic) for the treatment of the above conditions are based on antimycotics, antibacterials, corticosteroids, non steroidal antiinflammatories or xylocaine and derivatives thereof (when itching prevails), or antihistaminics (only orally). In episodic infections there is no need for a change in the therapy, because the advantages outweigh potential risks (side effects, undesired local toxicity reactions, sensitisation); instead, in recurring episodes prevention would undoubtedly be a successful approach.

### Disclosure of the invention

The present invention relates to topical vaginal compositions, effective in the treatment of the symptoms and progress of vaginosis, vaginitis and vulvo-vaginitis, in particular of the recurring episodes.

The compositions of the present invention contain:
a) *Zanthoxylum bungeanum* extract;
b) 18-beta glycyrrhetic acid;
c) *Matricaria camomilla* essential oil
d) *Melaleuca alternifolia* essential oil;
e) *Curcuma longa* or curcumin extract;
f) lactic or propionic acid.

More particularly, the compositions of the present invention contain:
a) active fraction obtained through extraction of *Zanthoxylum bungeanum;*
b) 18-beta glycyrrhetic acid from *Glycyrrhiza glabra* roots;
c) active fraction from *Matricaria chamomilla* essential oil;
d) active fraction from *Melaleuca alternifolia* essential oil;
e) active fraction from *Curcuma longa;*
f) lactic or propionic acid.

These compositions proved effective in reducing the clinical progression and possible recurring episodes of the above described disorders.

According to a preferred aspect of the present invention, the active fraction obtained through extraction of *Zanthoxylum bungeanum* is a standardized lipophilic extract containing about 30% alkylamides (hydroxy-alfa/beta/gamma-sanshooli), which is prepared by extraction and subsequent fractioning with hypercritical CO2.

According to a preferred aspect of the present invention, the active fraction of *Matricaria chamomilla* essential oil has a bisabolol titre ranging from 5 to 50%, more preferably of 45%.

According to a preferred aspect of the present invention, 18-beta glycyrrhetic acid from *Glycyrrhiza glabra* root and the active fraction from Curcuma longa are present as complexes with phospholipids (phytosome).

Generally speaking, a phytosome is a chemical entity formed through stoichiometric interaction, in an aprotic solvent, of a standardised polyphenolic fraction or of a purified component thereof with phospholipids extracted from soy (*Glycine max*). These phospholipids belong to the class of the distearoylphosphatidylcholines. Due to their chemico-physical, spectroscopical and biological characteristics, phytosomes can be considered as new chemical entities and, compared to the active ingredients in the free form, they have increased biological activity, improved absorption and prolonged action.

*Zanthoxylum bungeanum,* a herbal remedy prepared according to what disclosed in EP 1096944 and to the literature (Leung AY (1990). Chinese medicinals. pp.: 499-510. In: Janick J. Timber Press, Portland, OR; Mizutani K et al. (1988), Chem. Pharm. Bull. 36, 2362-2365; Cristoni A et al. (1999), Cosmetics. April 20-22, Paris, France; Yusuda J et al. (1982), Phytochemistry 21, 1295-1298; Bowen JM et al. (1996), AJVR: 57, 1239-1244), is a perennial plant belonging to the Rutaceae family and having its origins in Cina, wherein it is widely used as spice able to reduce the irritating properties of some foods and as medicinal plant thanks to its anti-itching properties.

A standardized lipophilic extract containing about 30% alkylamides (hydroxy-alfa/beta/gamma-sanshooli) was prepared by extraction and subsequent fractioning with CO2 in hypercritical phase. This extract was tested *in vitro* to confirm the anti-itching and "analgesic/anaesthetic-like" activity of the titred fraction, and subjected to toxicological studies (mutagenesis according to the Ames-test, oral toxicity, ocular irritation, irritation and cutaneous sensitisation) which demonstrated complete absence of toxicity. The product was also tested on volunteers, showing complete tolerability and absence of sensitization.

18-Beta-glycyrrhetic acid is a pentacyclic triterpene, which is obtained by extraction and hydrolysis of liquorice root (*Glycyrrhiza glabra*). When complexed in the phytosomal form the product shows high activity after local administration.

When administered topically, the phytosome of glycyrrhetic acid acts as potent anti-inflammatory agent through inhibition of tissutal 11-beta hydroxysteroidehydrogenase, in particular of the natural conversion of active cortisol to the inactive form: through this enzymatic interaction, glycyrrhetic acid prolongs the normal antiinflammatory activity of cortisol which is normally released in the tissues after inflammatory stimuli.

In experimental tests for local antinflammatory activity, the product inhibited the oedema induced in the animal's paw by inoculum of Croton oil with higher efficacy (about 90%) than that of common non-steroidal anti-inflammatories (in this case indomethacin). From this point of view, 18-beta glycyrrhetic acid can be considered the most potent topical antiinflammatory of natural origin.

Bisabolol is the active ingredient of common chamomile essential oil (*Matricaria chamomilla*), endowed with a well-known and proven antiphlogistic action on the skin, mouth and nose. The substance, capable of arresting the most common inflammatory cutaneous conditions, was tested, with positive results, in acute dermatosis, contact dermatitis, allergic exanthema and eczema, proving at the same time remarkably safe. At present, bisabolol is considered one of the safest active principles for topical use, especially for cutaneous inflammation.

*Melaleuca alternifolia* is a herbaceous perennial plant living in tropical climates and having a very fleshy rhizomatous root with orange colour. The extraction of *Melaleuca alternifolia* leaves and branches under steam current allows to obtain an essential oil endowed with remarkable antimicrobial, antiviral, antiprotozoarian and antimicotic properties. Its aromatic component, as well as the non-aromatic one, is endowed with a clinical action which is evident especially in the genitourinary apparatus, as demonstrated by the numerous clinical studies.

*Curcuma longa* is a herbaceous perennial plant with a typical yellow rhizoma, having its origin in south Asia, India and dell'Indonesia, also grown in Africa, in the Antilles and in Brazil, Haiti and Jamaica, which is used in Ayurvedic medicine as a general depurative, as a digestive remedy and for the treatment of fever, infections, dysentery, arthritis, icterus and various hepatic disorders. In Cinese medicine curcuma is used for the treatment of liver and cistifellea problems, haemorrhages, chest congestions and menstrual disorders, presence of blood in the urine, toothache, contusions and ulcers (for external use).

Curcumin, obtained from *Curcuma longa,* is a substance having very low toxicity and endowed with analgesic, antiarthritis, antinfiammatory (thanks to its ability of inhibiting lipo-oxygenase and the cycloxygenase), antioxidants, bactericide, choleretic, digestive, diuretic, hypotensive, insecticide, blushing and stimulating properties. The stimulating nervous activity is effective on the epidermidis, in particular on piliferous bulbs.

It is conveniently used in topical preparations, especially as phytosomial forms, for it is characterized by very low oral absorption. Moreover, it has shown antimutagenic, antioxidant and chemopreventive activity in more in vitro models. The most important activity is mainly evidenced on the phlogistic responses mediated by cytokines and TNF-alpha. The vaginal area, wherein local phlogosis is mediated by release of Th1- and Th2-type cytokines, is greatly advantaged by the administration of curcumin, which acts on both these cytokines.

As is known, normal and phyisiological vaginal secretion has an acid pH of about 4.5; due to the production of lactic acid from glycogen by Doderlein's bacillus (this term improperly identifies the mixture of pyogen lactobacilli localised in the vagina). A normal oestrogen level is necessary in order to ensure the presence of a sufficient amount of glycogen in the vagina; as a consequence, in those periods of life when oestrogen production is low (prepuberal or postmenopausal age), the vaginal pH is less acid and colonization by pathogenic germs is easier. Due to the fact that local therapy based on estrogens is not very practicable, said problem can be treated by topical use of lactic acid which is per se an effective physical barrier against pathogens colonisation, because it lowers the pH to values of about 4. The same action (although moderate), can be obtained by adding to the composition phytoestrogen aglycons produced by enzymatic or chemical hydrolysis, as already disclosed in MI2003A001535.

The compositions of the present invention (in which each ingredient has a dose comprised between 0.001 and 15%), which can be administered through the vaginal route, can be prepared according to conventional methods, well-known in the pharmaceutical technique, such as those described in "Remington's Pharmaceutical Handbook", Mack Publishing Co., N.Y., USA, together with suitable pharmaceutically acceptable excipients. Examples of compositions according to the invention are vaginal gels, creams, ointments, o/w emulsions, w/o emulsions, sprays, pessaries, suppositories, ovules and tablets, sterile gauzes or towels for cleansing the vaginal area and for intimate hygiene.

The examples reported below further illustrate the invention.

### Formulation examples

### EXAMPLE 1 - VAGINAL CREAM

| Ingredient | % Amount |
|---|---|
| *Zanthoxylum bungeanum* | 0.5% |
| 18-beta glycyrrhetic acid phytosome | 1.5% |
| Bisabolol | 0.1% |
| *Melaleuca alternifolia e.o.* | 0.25% |
| Curcumin phytosome | 0.25% |
| lactic acid | 1.0% |

### EXAMPLE 2 - VAGINAL GEL

| Ingredient | % Amount |
|---|---|
| *Zanthalene* | 1% |
| 18-beta glycyrrhetic acid | 1.0% |
| Bisabolol | 0.3% |
| *Melaleuca alternifolia e.o.* | 0.25% |
| *Curcuma longa* dry standardized extract | 0.1% |
| lactic acid | 1.0% |

### EXAMPLE 3 - VAGINAL OVOIDS

| Ingredient | % Amount |
|---|---|
| *Zanthoxylum bungeanum* | 1.5% |
| 18-beta glycyrrhetic acid phytosome | 1.5% |
| Bisabolol | 0.2% |
| *Melaleuca alternifolia e.o.* | 0.1% |
| Curcumin phytosome | 0.25% |
| lactic acid | 1.0% |

## Claims

1. Vaginal compositions for topical use containing:
a) *Zanthoxylum bungeanum* extract;
b) 18-beta glycyrrhetic acid;
c) *Matricaria chamomilla* essential oil;
d) *Melaleuca alternifolia* essential oil;
e) *Curcuma longa* or curcumin extract;
f) lactic or propionic acid.

2. Compositions as claimed in claim 1, containing:
a) an active fraction obtained through extraction of *Zanthoxylum bungeanum;*
b) 18-beta glycyrrhetic acid from *Glycyrrhiza glabra* root;
c) active fraction from *Matricaria chamomilla* essential oil;
d) active fraction from *Melaleuca alternifolia* essential oil;
e) active fraction from *Curcuma longa;*
f) lactic or propionic acid.

3. Compositions as claimed in claim 1 or 2, in which the active fraction of *Zanthoxylum bungeanum* is a standardized lipophilic extract containing about 30% alkylamides prepared by extraction and subsequent fractioning with hypercritical CO2.

4. Compositions according to any one of claims 1 to 3, in which the active fraction of *Matricaria chamomilla* essential oil has a bisabolol content between 5 and 50%, more preferably of 45%.

5. Compositions according to any one of claims 1 to 4, in which 18-beta glycyrrhetic acid from *Glycyrrhiza glabra* root and the active fraction from *Curcuma longa* are present as complexes with phospholipids (phytosome).

6. Compositions according to any one of claims 1 to 5, in the form of gels, creams, ointments, o/w emulsions, w/o emulsions, sprays, pessaries, suppositories, ovules and vaginal tablets, ganzes or sterile towels for the cleansing of the vaginal area and for the intimate hygiene.

7. Use of:
a) active fraction obtained through extraction of *Zanthoxylum bungeanum;*
b) 18-beta glycyrrhetic acid in the free form or as phytosome from *Glycyrrhiza glabra* root;
c) active fraction of *Matricaria chamomilla* essential oil;
d) active fraction of *Melaleuca alternifolia* essential oil;
e) active fraction from *Curcuma longa;*
f) actic or propionic acid;
for the preparation of compositions for the prevention and treatment of inflammatory and/or infective conditions of the genital area.

8. The use as claimed in claim 7, in which the inflammatory and/or infective conditions of the genital are vaginosis, vaginitis and vulvo-vaginitis.
